# EUROPEAN PATENT SPECIFICATION

(11) **EP 0 487 149 B1**
(45) Date of publication and mention of the grant of the patent: **05.03.1997**
(21) Application number: 91202977.4
(22) Date of filing: 15.11.1991
(51) Int. Cl.: G01N 35/00

(54) **Analyzers**
Testelementfördervorrichtung für Analysatoren
Mécanisme de transfert des éléments de test pour analyseurs

(30) Priority: 19.11.1990 US 615530
(43) Date of publication of application: 27.05.1992
(60) Divisional application: 94116615.9
(73) Proprietor: Johnson & Johnson Clinical Diagnostics, Inc., Rochester New York 14650 (US)
(72) Inventor: Shaw, James David, c/o EASTMAN KODAK COMPANY, Rochester, New York 14650-2201 (US); Muszak, Martin Frank, c/o EASTMAN KODAK COMPANY, Rochester, New York 14650-2201 (US)
(74) Representative: Mercer, Christopher Paul

(56) References cited:
- EP-A- 0 231 951
- EP-A- 0 301 743
- DE-C- 79 712
- DE-U- 8 804 264
- US-A- 4 224 032
- RESEARCH DISCLOSURE no. 197, September 1980, HAVANT GB pages 375 - 376 '19370: SLIDE TRANSFER APPARATUS'

## Description

This invention relates to analyzers for ascertaining analyte concentrations in body liquids which are dispensed on to test elements, and is particularly concerned with analyzers which need a wash station to allow immunoassays to be conducted.

Analyzer mechanisms have been provided for receiving slide test elements from incubators, to carry them on to additional stations, for example, a wash station. Such a mechanism is described in US-A-4 857 471. Although this mechanism functions admirably, it uses a platform which lowers into the "dump" path of the ejected slide element to catch the slide element. The platform cannot move on to the wash station but provides a stationary support. As a result, a claw must then be used to transfer the slide element from this stationary support to a movable train. Thus, the noted mechanism has the disadvantage of requiring a transfer claw and means other than the catching surface to move the slide element to the wash station. Furthermore, the train which is used for the wash step transfer is of substantial size and complexity.

It is therefore an object of the present invention to provide an analyzer with a transfer mechanism which allows a slide element to be removed from the analyzer and washed, and uses simpler and less expensive parts than the prior art mechanisms.

An analyzer has been constructed and method for operation thereof has been devised as defined in the claims which solve the aforesaid problems. The mechanism includes a catcher plate which provides the required movement of a "caught" test element ejected from one of the stations of the analyzer to a wash station. Furthermore, the catcher plate is constructed to provide other important features, all in one simplified piece.

More specifically, in accordance with one aspect of the present invention, there is provided an analyzer comprising:-
a plurality of stations which includes an incubator and a wash station positioned outside of the incubator,
ejecting means for ejecting a test element from one of the stations, and
discharge path defining means for defining a discharge path along which the ejected test element is discharged from the analyzer;
wherein the analyzer further includes a catcher plate for catching ejected test elements, and drive means for moving the plate into the discharge path to intercept an ejected test element, the drive means including track-defining means for moving the catcher plate and an intercepted test element from the discharge path to the wash station.

In accordance with a second aspect of the present invention, there is provided a method of washing an incubated test element comprising the steps of:-
loading a test element at a first station into an incubator,
ejecting at a second station, a distance away from the first station, a loaded test element from the incubator,
catching the ejected test element by inserting a plate into the path of the ejected test element,
shuttling the caught element to a wash station by moving the plate and its test element to the wash station,
washing the shuttled test element at the wash station, and
returning the washed element to the incubator by moving the plate and its test element back into the first station,
and reloading the washed element into the incubator.

Accordingly, it is an advantageous feature of this invention that the same means which catches a test element ejected from the incubator is used to transport such test element to a wash station.

It is a related advantageous feature of the invention that the means for transporting a test element from its ejected location to an additional station is simplified.

It is another advantageous feature of the invention that a removed and washed test element is returned to the original loading mechanism for reloading into the incubator to avoid the use of a separate loader.

For a better understanding of the present invention, reference will now be made, by way of example only, to the accompanying drawings in which:-
Figure 1 is a partially schematic, plan view of an analyzer constructed in accordance with the present invention;
Figure 2 is a fragmentary perspective view showing especially the shuttle apparatus of the present invention outside the incubator;
Figure 3 is a fragmentary side elevational view taken generally along the line III-III of Figure 1;
Figure 4 is a perspective view of the catcher plate which reciprocates within the shuttle apparatus shown in Figure 2;
Figure 5 is a plan view of the catcher plate shown in Figure 4, showing in phantom a test element E carried by it;
Figure 6 is a fragmentary, partially schematic (for element E) sectioned view taken generally along the line VI-VI of Figure 5;
Figure 7 is a fragmentary plan view of the shuttle apparatus shown in Figure 3;
Figures 8 and 9 are sectioned views taken generally along the lines VIII-VIII and IX-IX of Figure 7 respectively; and
Figures 10, 11, 12 and 13A to 13C are fragmentary sectioned views taken generally along the line Q-Q of Figure 7, illustrating the sequential operation of the shuttle apparatus.

The invention is hereinafter described in connection with the preferred embodiments, in which the transfer mechanism which catches and transfers slide-like test elements is disposed outside of an incubator particularly positioned in an analyzer, to transfer the test element to a wash station and back to the incubator, and in which the test elements are of a type similar to those obtained from Eastman Kodak Company under the trademark "Ektachem" slides, or from Fuji Photo under the tradename "Drychem".

Still further, such a transfer mechanism is useful regardless of the construction of the test element, although generally planar elements are preferred since the transfer mechanism is shaped preferably to handle such planar elements.

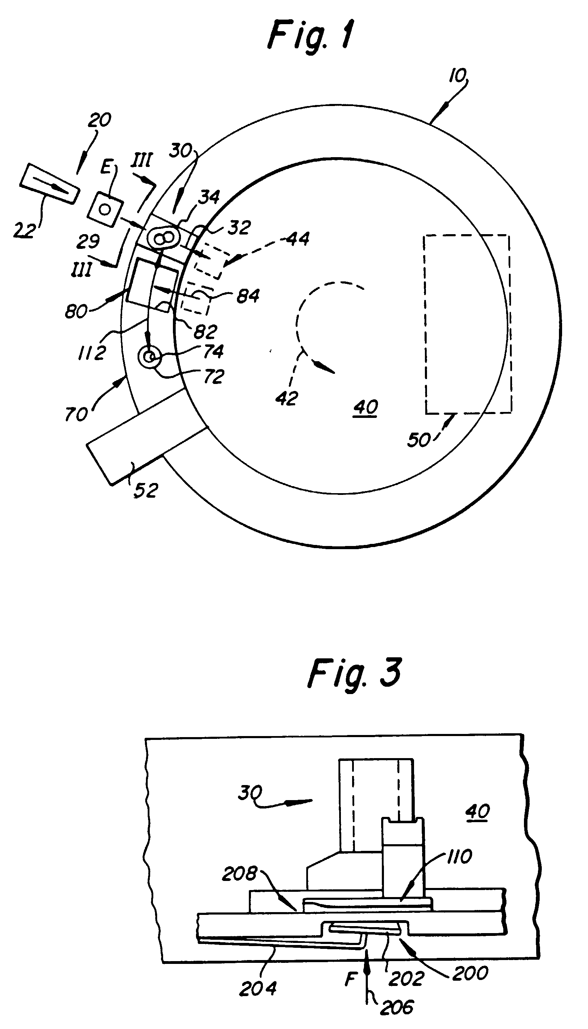

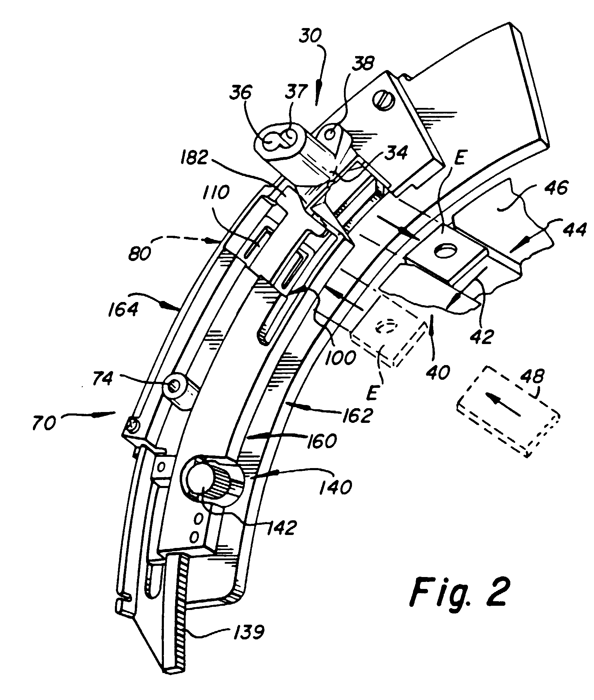

An analyzer 10 in which this shuttle invention is useful is shown in Figure 1 and preferably comprises a station 20 for loading a slide-like test element E into a sample dispensing station 30, and for loading such an element, along path 32, now bearing patient sample, into an incubator 40. Preferably, loading station 20 includes a pusher blade 22 which pushes an element E along path 29 so as to be injected into station 30. The loading station includes tip locator 34, as shown more clearly in Figure 2, with two apertures 36, 37 as is conventional for patient sample metering, and an aperture 38 for reference liquid metering, as is also conventional. Also preferably, the incubator 40 is the rotating type, rotating in the direction of arrow 42, and includes a reflectometer 50 (Figure 1) for scanning colorimetric test elements while they are held at a plurality of stations 44, etc. (Figure 2) as defined by a rotor 46.

Such an analyzer includes an electrometer 52, as shown in Figure 1, for reading potentiometric test elements after they are removed from the incubator by, for example, a pusher blade 48 shown in Figure 2. A wide variety of incubators is useful for this purpose, for example, that disclosed for example in US-A-4 935 374.

An analyzer construction similar to that described in US-A-4 857 471 is shown in Figure 2. A wash station 70 is disposed outside of incubator 40 adn is displaced circumferentially from sample dispensing station 30. The wash station 70 comprises a boss 72 and aperture 74 which serve to hold a dispensing tip (not shown) in proper orientation with respect to a test element to be washed. In between stations 30 and 70 is an eject station 80, including a discharge path defined by aperture 82 (Figure 1) into which a test element is ejected, in the direction shown by arrow 84, when its readings are completed. Shuttle apparatus is then provided to allow test elements to be intercepted at station 80, taken to wash station 70, and reinserted into the incubator, as described in US-A-4 857 471. In accordance with one aspect of the invention, it is the improvement of this apparatus to which the invention is addressed.

More specifically, the shuttle apparatus 100, shown in Figure 2, comprises a catcher plate 110, means 160 for supporting catcher plate 110 for movement along a path indicated by arrow 112 in Figure 1 which is preferably curvilinear, and means 140 for driving plate 110 along path 112. Importantly, path 112 is constructed to extend back to station 30 to intersect path 32 so that a test element washed at station 70 can be reinserted into the loading path 32.

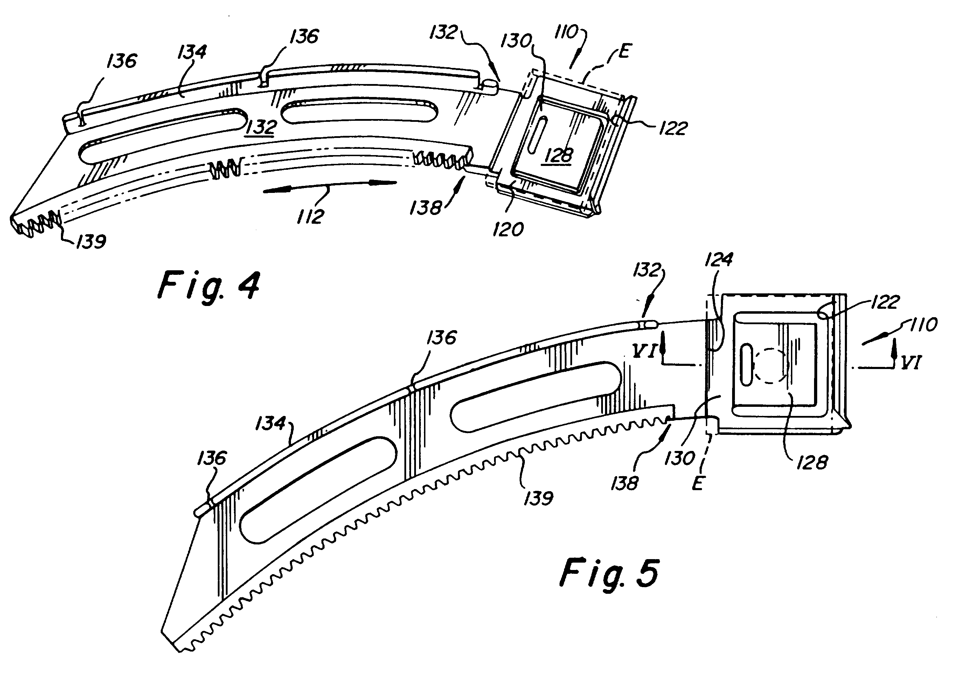

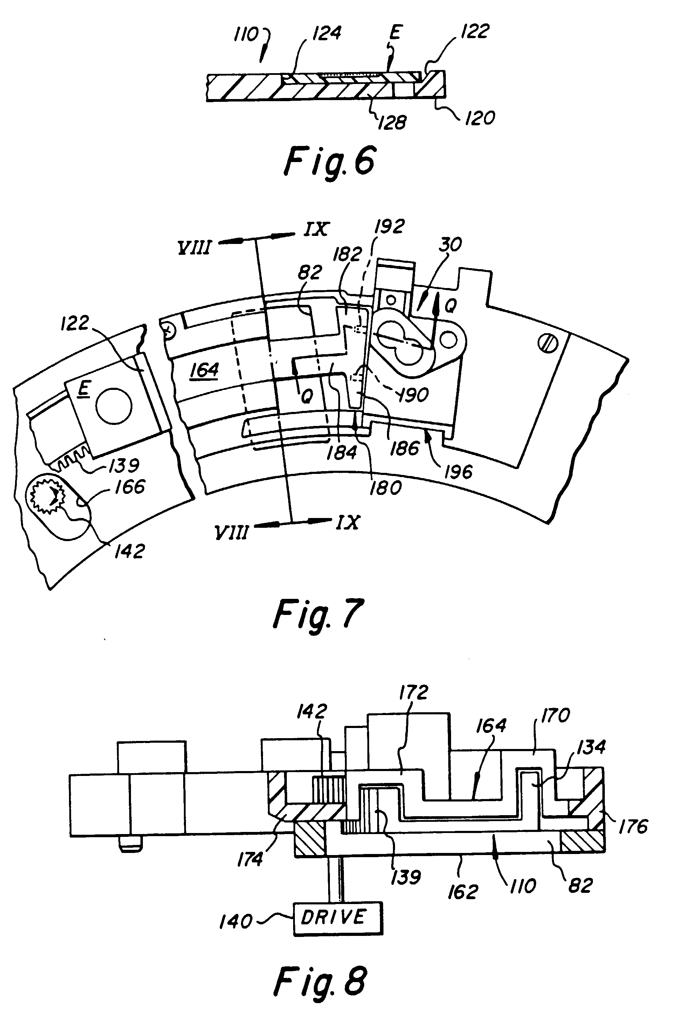

Referring now to Figures 4 to 6, catcher plate 110 comprises a frame 120 shaped to hold a test element E, shown in phantom. Accordingly, frame 120 is generally rectangular, and is provided with two opposed shoulders 122, 124 shaped and positioned, as is shown more clearly in Figure 6, to restrain element E from moving off plate 110 as the latter moves on path 112 (Figure 4). Shoulder 122 is the leading shoulder and is preferably beveled, to allow shoulder 122 to cam under element E when the latter is returned to and retained at path 32 (Figure 1) as described hereinafter.

A central support member 128 is flexibly connected to frame 120 to do the principal carrying of element E. The flexibility is achieved by reason of the cantilever connection of support member 128 at one side 130 of frame 120. As a result, member 128 is able to flex relative to frame 120, in and out of the plane defined by frame 120.

Plate 110 is preferably integrally connected to a drive tongue 132 which extends along a curvilinear arc which matches the curve of means 160 and path 112. The outside edge of tongue 132 has a raised ridge 134 provided with means, such as slots 136, to cooperate with a sensor. The inside edge 138 of tongue 132 comprises a raised ridge which is provided with a rack 139. Rack 139 is driven by gear 142 of drive means 140 shown in Figure 2.

Support means 160 for plate 110 and its tongue 132 comprises two opposed track members 162 and 164 as shown in Figures 7 to 9, between which plate 110 and tongue 132 reciprocate. Members 162 and 164 preferably have the same arcuate curvature as tongue 132. Most preferably, member 162 is generally flat (Figure 8) and is apertured at 82 for element discharge, and at 166 to receive drive gear 142 (Figure 7). Opposed track member 164 is rail-shaped at 170, 172 to accommodate ridge 134, and rack 139 of tongue 132 (Figure 8). Member 164 is secured to lower member 162 at bottom portions 174 and 176. Member 164 is apertured to accommodate gear 142, and further at 74, as shown in Figures 1 and 2, to provide for wash station 70.

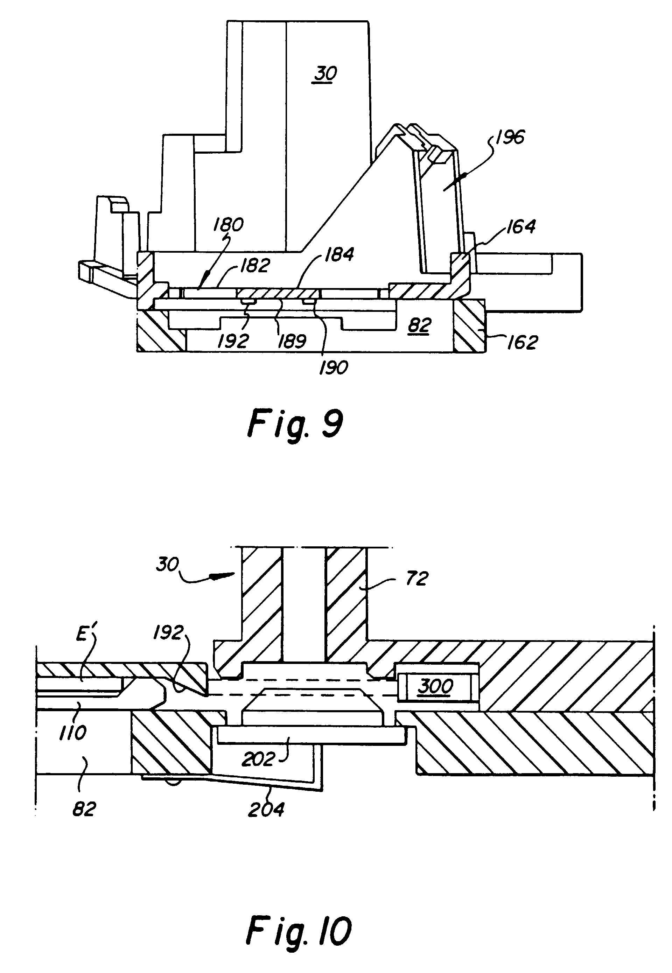

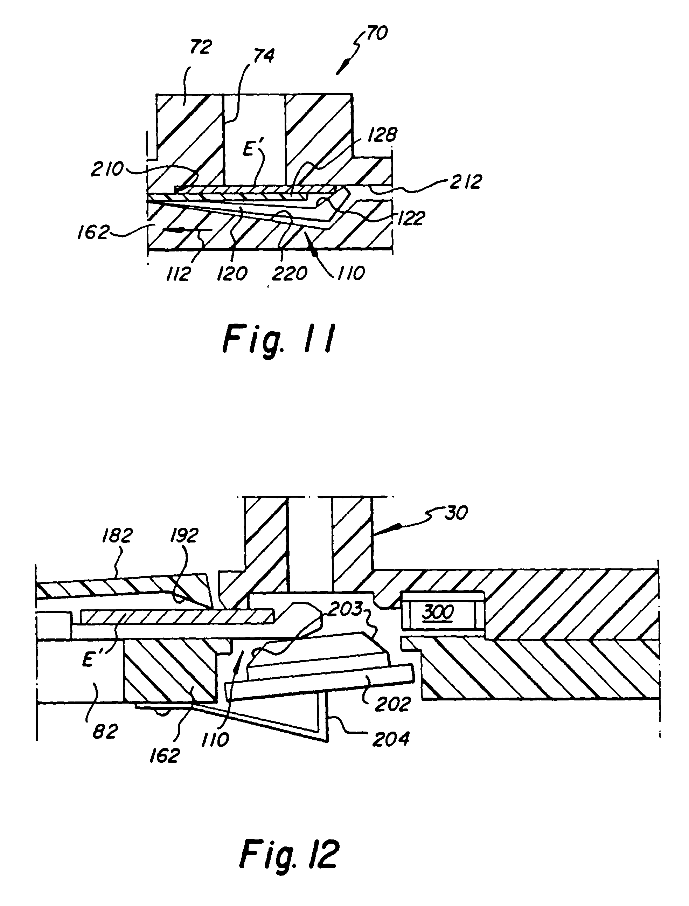

In another aspect of the invention, there is provided stop means 180 which allow a washed test element to be returned and retained at station 30 (Figure 7). For this purpose, stop means 180 is disposed adjacent the injection path 29, 32, at the intersection location of that path with path 112. Most preferably, stop means 180 comprise a flexure plate 182, as shown in Figures 2 and 7, which is cantilevered by arm 184 from the rest of upper member 164. The outer edge 186 of plate 182 provides a shoulder against which a test element abuts, when it moves along path 29, 32. In addition, flexure plate 182 includes on its undersurface 189, as shown in Figure 9, one and preferably two camming feet 190, 192, which allow plate 182 to ride up over a test element, as shown in Figure 12, being moved by plate 110 on path 112 to path 29, 32.

Optionally, a viewing port 196 can be provided, as shown in Figure 4, adjacent station 30 to allow a wetness detector to scan a slide element as liquid is dispensed thereon.

The apparatus of the invention further includes bias means 200 at station 30, as shown more clearly in Figure 3, and locating surfaces 210, 212, as shown more clearly in Figure 11, at wash station 70. In station 30, the bias means 200 acts to bias a test element up against the tip locator 34 at station 30. Means 200 comprise a platen 202 which is beveled at 203 (Figure 12) and a spring 204 exerting an upward force F, in the direction of arrow 206 in Figure 3. Entrance slot 208 allows a test element to be inserted into station 30 and on to either platen 202 or shuttle plate 110.

At station 70 (Figure 11), stop surface 210 is provided to stop the movement of a test element E' even as plate 110 continues to advance slightly further, in the direction of arrow 112. Undersurface 212 at station 70 is the ceiling against which element E' is pushed by flexible support member 128. An opposite depression 220 is formed in lower track member 162 to receive frame 120 of plate 110, which is cammed downwardly due to camming surface 122 of frame 120 pressing against element E'. In addition, a camming surface, not shown, extending diagonally from surface 210 ensures proper location of element E' in the direction out of the plane of Figure 11.

The wash method will be readily apparent from the previous description. In brief, plate 110 is moved by drive means 140 into a position so as to intercept an ejected test element E' (Figure 10) thus preventing element E' from falling out discharge aperture 82.

Next, plate 110 moves along path 112 due to the action of drive means 140, until element E' is at wash station 70 (Figure 11). A suitable pipette, not shown, is inserted into aperture 74, and boss 72 serves to hold the pipette the proper distance within station 70. At the same time, plate 110 pulls element E' up against stop shoulder 210 and the flexure of support member 128 is such as to push element E' up against undersurface 212 of station 70. The proper spacing of the pipette and element E' is now defined, which can be, for example, about 1.3mm. About 10µl of wash liquid is preferably ejected on to the element E', preferably at a rate of about 0.5µl/s, for 20s. However, other rates can also be used, depending on the hydrophilicity of the element being washed.

After washing, plate 110 is now returned towards station 30 and away from station 70, by reversing the direction of rotation of gear 142.

In accordance with another aspect of the present invention, the wash method differs from that previously used in that the washed element is returned to the station from which elements that have just received sample are loaded into the incubator. This allows the analyzer to be simplified in that the same pusher blade used to initially load the element into the analyzer is re-used to re-load the element. More specifically, as plate 110 and element E' move from the vicinity of discharge path 82 into station 30 where path 112 intersects path 29, 32 (Figure 12) camming surfaces 190 and 192 allow flexure plate 182 of stop means 180 to ride up over element E'. At the same time, platen 202 is cammed downwardly due to the camming action caused by surface 203.

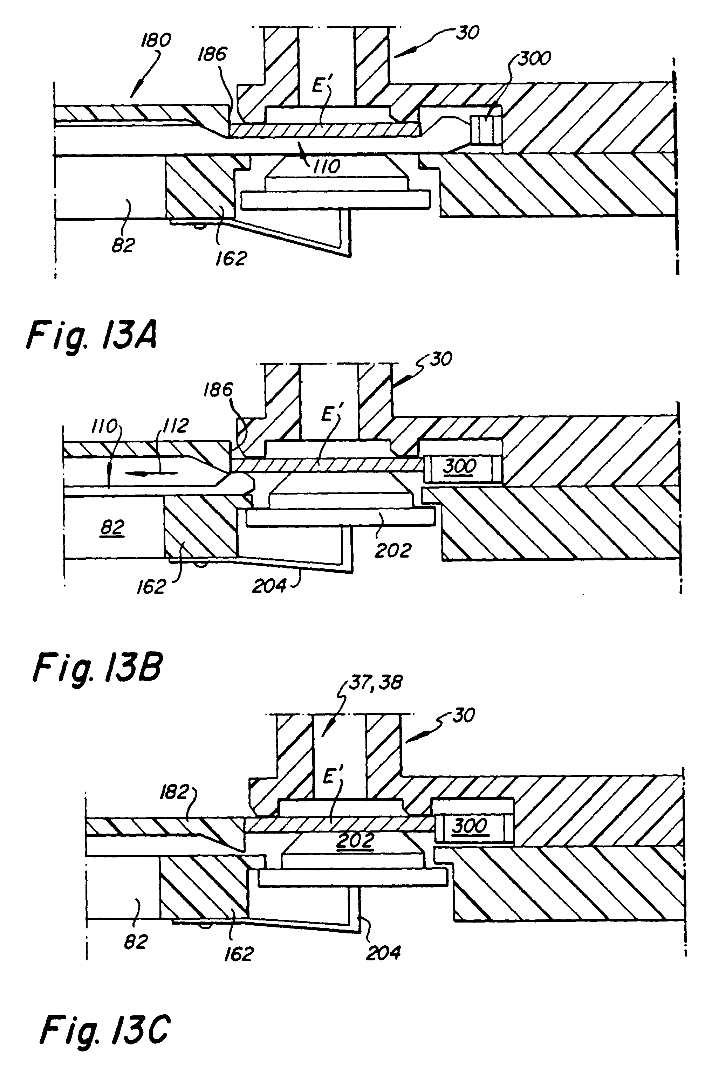

Once element E' is returned to station 30, as shown in Figures 13A to 13C, stop means 180 is effective to restrain element E' from leaving station 30 with plate 110. That is, shoulder 186 slips behind element E' (Figure 13A) so that as plate 110 starts moving out of station 30 along the path of arrow 112 (Figure 13B) shoulder 186 holds element E' from following plate 110. Plate 110 is carefully advanced into the position shown in Figure 13A by drive means 140 to ensure element E' is advanced past shoulder 186. The steps of travel of means 140 can be adjusted to ensure that this advance occurs. Meanwhile, platen 202 is pushed up by its spring 204 to further hold element E'. When plate 110 is completely withdrawn (Figure 13C), element E' is positioned for re-loading into incubator 40 using pusher blade 22. (The positioning of the parts in Figure 13C is also their position when an element is first loaded into station 30 for dispensing patient sample and/or reference liquid via apertures 36, 37 and 38, of which 38 is not shown.)

A bumper spring 300 is preferably included, as shown in Figure 13A, against which plate 110 pushes when element E' is being returned to station 30. This spring prevents over-travel of plate 110, but primarily it assists in holding test elements against stop shoulder 186 (Figure 13B).

Following re-loading of the washed slide into the incubator which occurs after the events illustrated in Figure 13C, further incubation and a reading of the element occur. When a read element is ready for disposal, ejection occurs using pusher blade 48, operating in the direction of arrow 310 in Figure 2, except this time, plate 110 is not in position at station 80 to catch the element. Instead, the element falls through aperture 82 (Figure 1) into a suitable disposal container.

## Claims

1. An analyzer (10) comprising:-
a plurality of stations (20, 30, 40, 50, 52, 70, 80) which includes an incubator (40) and a wash station (70) positioned outside of the incubator (40),
ejecting means (48) for ejecting a test element (E) from one of the stations (20, 30, 40, 50, 52);
discharge path defining means (82) for defining a discharge path along which the ejected test element (E) is discharged from the analyzer (10); and a catcher plate (110, 120, 122, 124, 128, 130) for catching ejected test elements (E),
characterized in that the analyzer further includes drive means (132, 139, 140, 142, 160, 162, 164, 170, 172) for moving the plate (110, 120, 122, 124, 128, 130) into the discharge path (82) to intercept an ejected test element (E), the drive means (132, 139, 140, 142, 160, 162, 164, 170, 172) including track-defining means (160, 162, 164) for moving the catcher plate (110, 120, 122, 124, 128, 130) and an intercepted test element (E) from the discharge path (82) to the wash station (70).

2. An analyzer according to claim 1, wherein the catcher plate (110, 120, 122, 124, 128, 130) comprises a frame (120) having opposed, raised shoulders (122, 124) dimensioned to retain a test element (E) therebetween to prevent the test element (E) being displaced.

3. An analyzer according to claim 2, wherein the catcher plate further includes a central support member (128) disposed between the shoulders (122, 124) and flexibly connected to the frame (120) to allow relative flexing between the central support member (128) and the frame (120) in and out of the plane of the frame (120).

4. An analyzer according to claim 3, wherein the central support member (128) is cantilevered from the frame (120) at only one side (130) thereof to provide the relative flexing.

5. An analyzer according to any one of claims 2 to 4, wherein the shoulders (122, 124) are respectively disposed on the leading and trailing edges of the plate (110, 120, 122, 124, 128, 130) as it is moved along the track (160, 162, 164) from the wash station (70), the leading edge shoulder (122) being provided with a cam surface sloped sufficiently to allow the frame (120) to ride under a test element (E') which is on the plate (110, 120, 122, 124, 128, 130) if the test element (E') is held from movement along the track (160, 162, 164) when the frame (120) is moved.

6. An analyzer according to any one of the preceding claims, further including inject path defining means for defining an inject path (29, 32) for injecting a test element (E) into a station (30).

7. An analyzer according to claim 6, wherein the inject path (29, 32) is disposed to intersect the track means (160, 162, 164) at an intersect location, and stop means (180, 182, 184, 186, 190, 192) are included adjacent the inject path (29, 32) and the intersect location for preventing a test element (E) at that location from being moved off the inject path (29, 32).

8. An analyzer according to claim 7, wherein the stop means (180, 182, 184, 186, 190, 192) is flexibly attached to the track defining means (160, 162, 164) and further includes a camming surface (190, 192) shaped to allow the stop means (180, 182, 184, 186, 190, 192) to ride up over a test element (E) being moved by the catcher plate (110, 120, 122, 124, 128, 130).

9. An analyzer according to any one of claims 6 to 8, further including bias means (200, 202, 203, 204, 208) below the inject path defining means for biasing a portion (128) of the plate (110, 120, 122, 124, 128, 130) upwardly it is moved by the drive means (132, 139, 140, 142, 160, 162, 164, 170, 172) into the intersect location.

10. An analyzer according to any one of the preceding claims, wherein the track-defining means (160, 162, 164) include a bottom member and a cover plate, the cover plate and member being spaced vertically apart a distance sufficient to accommodate the catcher plate (110, 120, 122, 124, 128, 130), the cover plate including, at the wash station, restraining means (300) for catching and holding a test element (E) against further movement along the track away from the discharge path (82), and wherein the track-defining means (160, 162, 164) and the drive means (132, 139, 140, 142, 160, 164, 170, 172) are constructed to move the plate (110, 120, 122, 124, 128, 130) farther away from the discharge path (82) than where the restraining means (300) catches a test element (E), so that the leading edge cam surface forces a test element (E) against the cover plate.

11. An analyzer (10) comprising an incubator (40), inject path defining means for injecting a test element (E) containing a sample liquid along an inject path (29, 32) into the incubator (40), discharge path defining means for defining a discharge path (82) from the analyzer for receiving test elements (E) ejected from the incubator (40), a processing station (70) exterior to the incubator (40), intercepting means (110, 120, 122, 124, 128, 130) for intercepting a test element (E) ejected along the discharge path (82), and drive means (132, 139, 140, 142, 160, 162, 164, 170, 172) for moving an intercepted test element (E) along a track between the discharge path (82) and the exterior processing station (70),
characterised in that the intercepting means (110, 120, 122, 124, 128, 130) comprises a central support member (128) disposed between two shoulders (122, 124) and flexibly connected to a frame (120) to allow relative flexing between the central support member (128) and the frame (120) in and out of the plane of the frame (120), and wherein the drive means (132, 139, 140, 142, 160, 162, 164, 170, 172) comprise a drive (140) for reciprocating support member (128,122,124) along the track between the discharge path (82) and the processing station (70).

12. An analyzer according to any one of the preceding claims, wherein the track-defining means (160, 162, 164) are arcuately shaped to define an arcuate track.

13. A method of washing an incubated test element (E) comprising the steps of:-
loading a test element at a first station (20) into an incubator,
ejecting at a second station (80), a distance away from the first station, a loaded test element from the incubator,
catching the ejected test element by inserting a plate (110,120,122,124,128,130) into the path of the ejected test element,
shuttling the caught element to a wash station (70) by moving the plate and its test element to the wash station,
washing the shuttled test element at the wash station, and
returning the washed element to the incubator by moving the plate and its test element back into the first station,
and reloading the washed element into the incubator.

## Patentansprüche

1. Analysegerät (10) mit
einer Anzahl von Stationen (20, 30, 40, 50, 52, 70, 80) einschließlich eines Inkubators (40) und einer Waschstation (70) außerhalb des Inkubators (40);
einer Abgabeeinrichtung (48) zur Abgabe eines Testelements (E) aus einer der Stationen (20, 30, 40, 50, 52);
einer Abgabeweg-Festlegungseinrichtung (82) zum Festlegen eines Abgabewegs, längs dem das abgegebene Testelement (E) vom Analysegerät (10) abgegeben wird; und mit
einer Fangplatte (110, 120, 122, 124, 128, 130) zur Aufnahme von abgegebenen Testelementen (E);
dadurch **gekennzeichnet**, daß das Analysegerät des weiteren eine Antriebseinrichtung (132, 139, 140, 142, 160, 162, 164, 170, 172) zum Bewegen der Platte (110, 120, 122, 124, 128, 130) in den Abgabeweg (82) aufweist, um ein abgegebenes Testelement (E) aufzunehmen, wobei die Antriebseinrichtung (132, 139, 140, 142, 160, 162, 164, 170, 172) eine eine Bahn festlegende Einrichtung (160, 162, 164) zum Bewegen der Fangplatte (110, 120, 122, 124, 128, 130) und eines aufgenommenen Testelements (E) vom Abgabeweg (82) zur Waschstation (70) umfaßt.

2. Analysegerät nach Anspruch 1, wobei die Fangplatte (110, 120, 122, 124, 128, 130) einen Rahmen (120) mit gegenüberliegenden erhöhten Schultern (122, 124) aufweist, die so bemessen sind, daß dazwischen ein Testelement (E) festgehalten wird, um zu verhindern, daß sich das Testelement (E) verschiebt.

3. Analysegerät nach Anspruch 2, wobei die Fangplatte ein mittleres Halteelement (128) aufweist, das zwischen den Schultern (122, 124) angeordnet und flexibel mit dem Rahmen (120) verbunden ist, um eine relative Biegung gegenüber dem mittleren Halteelement (128) und dem Rahmen (120) in und aus der Ebene des Rahmens (120) zu ermöglichen.

4. Analysegerät nach Anspruch 3, wobei das mittlere Halteelement (128) am Rahmen (120) an nur einer Seite (130) befestigt ist, um die relative Biegung zu ermöglichen.

5. Analysegerät nach einem der Ansprüche 2 bis 4, wobei die Schultern (122, 124) am vorderen bzw. hinteren Rand der Platte (110, 120, 122, 124, 128, 130) angeordnet sind, wenn diese sich längs der Bahn (160, 162, 164) von der Waschstation (70) weg bewegt, wobei die Schulter (122) am vorderen Rand mit einer Nockenfläche versehen ist, die ausreichend geneigt ist, damit der Rahmen (120) unter ein Testelement (E') gleitet, das sich auf der Platte (110, 120, 122, 124, 128, 130) befindet, wenn das Testelement (E') von einer Bewegung längs der Bahn (160, 162, 164) abgehalten wird, wenn sich der Rahmen (120) bewegt.

6. Analysegerät nach einem der vorstehenden Ansprüche, mit einer Eingabeweg-Festlegungseinrichtung zum Festlegen eines Eingabeweges (29, 32) zur Eingabe eines Testelements (E) in eine Station (30).

7. Analysegerät nach Anspruch 6, wobei der Eingabeweg (29, 32) so angeordnet ist, daß die Bahneinrichtung (160, 162, 164) an einer Schnittstelle geschnitten wird, wobei angrenzend an den Eingabeweg (29, 32) und der Schnittstelle ein Anschlag (180, 182, 184, 186, 190, 192) vorgesehen ist, um zu verhindern, daß ein Testelement (E) an dieser Stelle vom Eingabeweg (29, 32) weg bewegt wird.

8. Analysegerät nach Anspruch 7, wobei der Anschlag (180, 182, 184, 186, 190, 192) flexibel an der Bahn-Festlegungseinrichtung (160, 162, 164) angebracht ist und eine Nockenfläche (190, 192) aufweist, die so geformt ist, daß der Anschlag (180, 182, 184, 186, 190, 192) über ein Testelement (E) gleitet, das von der Fangplatte (110, 120, 122, 124, 128, 130) bewegt wird.

9. Analysegerät nach einem der Ansprüche 6 bis 8, mit einer Vorspannungseinrichtung (200, 202, 203, 204, 208) unter der Eingabeweg-Festlegungseinrichtung, um einen Teil (128) der Platte (110, 120, 122, 124, 128, 130) nach oben vorzuspannen, wenn sie von der Antriebseinrichtung (132, 139, 140, 142, 160, 162, 164, 170, 172) zur Schnittstelle bewegt wird.

10. Analysegerät nach einem der vorstehenden Ansprüche, wobei die Bahn-Festlegungseinrichtung (160, 162, 164) ein Bodenelement und eine Abdeckplatte, wobei die Abdeckplatte und das Bodenelement vertikal einen Abstand aufweisen, der ausreicht, die Fangplatte (110, 120, 122, 124, 128, 130) einschließlich der Abdeckplatte an der Waschstation aufzunehmen, und eine Halteeinrichtung (300) zum Erfassen und Festhalten eines Testelements (E) gegen eine weitere Bewegung längs der Bahn vom Abgabeweg (82) weg umfaßt, und wobei die Bahn-Festlegungseinrichtung (160, 162, 164) und die Antriebseinrichtung (132, 139, 140, 142, 160, 164, 170, 172) so aufgebaut sind, daß die Platte (110, 120, 122, 124, 128, 130) vom Abgabeweg (82) weiter weg bewegt wird als an die Stelle, an der die Halteeinrichtung (300) ein Testelement (E) ergreift, so daß die Nockenfläche am vorderen Rand das Testelement (E) gegen die Abdeckplatte drückt.

11. Analysegerät (10) mit einem Inkubator (40), einer Eingabeweg-Festlegungseinrichtung zur Eingabe eines Testelements (E) mit einer Probenflüssigkeit längs eines Eingabeweges (29, 32) in den Inkubator (40), einer Abgabeweg-Festlegungseinrichtung zum Festlegen eines Abgabewegs (82) vom Analysegerät zur Aufnahme von Testelementen (E), die vom Inkubator (40) abgegeben werden, einer Bearbeitungsstation (70) außerhalb des Inkubators (40), einer Auffangeinrichtung (110, 120, 122, 124, 128, 130) zum Auffangen eines Testelements (E), das längs des Abgabeweges (82) abgegeben wird, und mit einer Antriebseinrichtung (132, 139, 140, 142, 160, 162, 164, 170, 172) zum Bewegen eines aufgefangenen Testelements (E) längs einer Bahn zwischen dem Abgabeweg (82) und der außerhalb liegenden Bearbeitungsstation (70)
dadurch **gekennzeichnet**, daß die Auffangeinrichtung (110, 120, 122, 124, 128, 130) ein mittleres Halteelement (128) aufweist, das zwischen zwei Schultern (122, 124) angeordnet und flexibel mit einem Rahmen (120) verbunden ist, um eine relative Biegung gegenüber dem mittleren Halteelement (128) und dem Rahmen (120) in und aus der Ebene des Rahmens (120) zu ermöglichen, wobei die Antriebseinrichtung (132, 139, 140, 142, 160, 162, 164, 170, 172) einen Antrieb (140) zum Hin- und Herbewegen des Halteelements (128, 122, 124) längs der Bahn zwischen dem Abgabeweg (82) und der Bearbeitungsstation (70) umfaßt.

12. Analysegerät nach einem der vorstehenden Ansprüche, wobei die Bahn-Festlegungseinrichtung (160, 162, 164) bogenförmig geformt ist, um eine bogenförmige Bahn festzulegen.

13. Verfahren zum Waschen eines inkubierten Testelements (E), mit den Schritten
des Eingebens eines Testelements an einer ersten Station (20) in einen Inkubator,
der Abgabe eines eingegebenen Testelements aus dem Inkubator an einer zweiten Station (80), die von der ersten Station einen bestimmten Abstand hat,
des Aufnehmens des abgegebenen Testelements durch Einfügen einer Platte (110, 120, 122, 124, 128, 130) in den Weg des abgegebenen Testelements,
des Beförderns des erfaßten Testelements zu einer Waschstation (70) durch Bewegen der Platte mit dem Testelement zu der Waschstation,
des Waschens des beförderten Testelements an der Waschstation, und
des Zurückbringens des gewaschenen Elements zum Inkubator durch Zurückbewegen der Platte mit dem Testelement in die erste Station,
und dem Zurückbringen des gewaschenen Elements in den Inkubator.

## Revendications

1. Analyseur (10) comprenant :
une pluralité de postes (20, 30, 40, 50, 52, 70, 80) qui incluent un incubateur (40) et un poste de lavage (70) disposé à l'extérieur de l'incubateur (40);
des moyens d'éjection (48) pour éjecter un élément de test (E) hors de l'un des postes (20, 30, 40, 50, 52);
des moyens (82) définissant un trajet d'évacuation, le long duquel l'élément de test éjecté (E) est évacué de l'analyseur (10); et
une plaque de saisie (110, 120, 122, 124, 128, 130) servant à saisir des éléments de test éjectés (E);
caractérisé en ce que l'analyseur transporte en outre des moyens d'entraînement (132, 139, 140, 142, 160, 162, 164, 170, 172) pour amener la plaque (110, 120, 122, 124, 128, 130) dans le trajet d'évacuation (82) pour intercepter un élément de test éjecté (E), les moyens d'entraînement (132, 139, 140, 142, 160, 162, 164, 170, 172) comprenant des moyens (160, 162, 164) définissant une piste et servant à déplacer la plaque de saisie (110, 120, 122, 124, 128, 130) et un élément de test intercepté (E) depuis le trajet d'évacuation (82) jusqu'au poste de lavage (70).

2. Analyseur selon la revendication 1, dans lequel la plaque de saisie (110, 120, 122, 124, 128, 130) comprend un cadre (120) comportant des épaulements surélevés opposés (122, 124), dimensionnés de manière à retenir un élément de test (E) entre eux pour empêcher que l'élément de test (E) soit déplacé.

3. Analyseur selon la revendication 2, dans lequel la plaque de saisie comporte en outre un élément de support central (128) disposé entre les épaulements (122, 124) et raccordés, de manière flexible, au cadre (120) pour permettre une flexion relative entre l'élément de support central (128) et le cadre (120) dans et hors du plan du cadre (120).

4. Analyseur selon la revendication 3, dans lequel l'élément de support central (128) s'étend en porte-à-faux à partir du cadre (120) uniquement d'un côté (130) de ce dernier de manière à permettre une section relative.

5. Analyseur selon l'une quelconque des revendications 2 à 4, dans lequel les épaulements (122, 124) sont disposés respectivement sur les bords avant et arrière de la plaque (110, 120, 122, 124, 128, 130) lorsqu'elle se déplace le long de la piste (150, 162, 164) à partir du poste de lavage (70), l'épaulement (122) du bord avant étant pourvu d'une surface de came suffisamment inclinée pour permettre au cadre (120) de venir au-dessous d'un élément de test (E'), qui est situé sur la plaque (110, 120, 122, 124, 128, 130) si l'élément de test (E') est empêché de se déplacer le long de la piste (160,162,164) lorsque le cadre (120) est déplacé.

6. Analyseur selon l'une quelconque des revendications précédentes, comprenant en outre des moyens définissant un trajet d'injection servant à définir un trajet d'injection (29,32) pour l'injection d'un élément de test (E) dans un poste (30).

7. Analyseur selon la revendication 6, dans lequel le trajet d'injection (29,32) est disposé de manière à intercepter les moyens formant piste (160, 162, 164) dans une position d'intersection, et des moyens d'arrêt (180, 182, 184, 186, 190, 192) sont prévus au voisinage du trajet d'injection (29, 32) et de l'emplacement d'intersection pour empêcher qu'un élément de test (E) présent en cet emplacement soit écarté du trajet d'injection (29, 32).

8. Analyseur selon la revendication 7, dans lequel les moyens d'arrêt (180, 182, 184, 186, 190, 192) sont fixés de manière flexible aux moyens (160,162,164) définissant la piste et comprennent en outre une surface à effet de came (190, 192) conformée de manière à permettre aux moyens d'arrêt (180,182,184,186,190,192) de venir au-dessus d'un élément de test (E) qui est déplacé par la plaque de saisie (110, 120, 122, 124, 128, 130).

9. Analyseur selon l'une quelconque des revendications 6 à 8, comprenant en outre des moyens de sollicitation (200, 202, 203, 204, 208) situés au-dessous des moyens définissant le trajet d'injection, pour solliciter une partie (122) de la plaque (110, 122, 124, 128, 130) vers le haut lorsqu'elle est amenée par les moyens d'entraînement (132, 139, 140, 142, 160, 162, 164, 170, 172) à l'emplacement d'intersection.

10. Analyseur selon l'une quelconque des revendications précédentes, dans lequel les moyens (160, 162, 164) définissant la piste comprennent un élément inférieur et une plaque de recouvrement, la plaque de recouvrement et l'élément étant séparés verticalement par une distance suffisante pour loger la plaque de saisie (110, 120, 122, 124, 128, 130), la plaque de recouvrement comprenant, au niveau du poste de lavage, des moyens de retenue (300) pour saisir et retenir l'élément de test (E) contre un déplacement supplémentaire le long de la piste, à partir du trajet d'évacuation (82), et dans lequel les moyens (160, 162, 164) définissant la piste et les moyens d'entraînement (132, 139, 140, 142, 160, 162, 164, 170, 172) sont agencés de manière à écarter la plaque (110, 120, 122, 124, 126, 130) à partir du trajet d'évacuation (82) plus loin que les moyens de retenue (300) saisissent un élément de test (E), de sorte que la surface de came du bord avant repousse un élément de test (E) contre la plaque de recouvrement.

11. Analyseur (10) comprenant un incubateur (40), des moyens définissant un trajet d'injection pour l'injection d'un élément de test (E) contenant un échantillon liquide le long d'un trajet d'injection (29, 32) dans l'incubateur (40), des moyens définissant un trajet d'évacuation pour définir un trajet d'évacuation (82) partant de l'analyseur, pour recevoir des éléments de test (E) éjectés de l'incubateur (40), un poste de traitement (70) situé à l'extérieur de l'incubateur (40), des moyens d'interception (110, 120, 122, 124, 128, 130) pour intercepter un élément de test (E) éjecté le long du trajet de décharge (82), et des moyens d'entraînement (132, 139, 140, 142, 160, 162, 164, 170, 172) servant à déplacer un élément de test intercepté (E) le long d'une piste entre le trajet d'évacuation (82) et le poste extérieur de traitement (110),
caractérisé en ce que les moyens d'interception (110, 120, 122, 124, 128, 130) comprennent un élément de support central (128) disposé entre deux épaulements (122, 124) et connecté de façon flexible à un cadre (120) de manière à permettre une flexion relative entre l'élément de support central (128) et le cadre (120) dans et hors du plan duc cadre (120), et dans lequel les moyens d'entraînement (132, 139, 140, 142, 160, 162, 164, 170, 172) comprennent une unité d'entraînement (140) pour déplacer en va-et-vient l'élément de support (128, 122, 124) le long de la piste entre le trajet d'évacuation (82) et le poste de traitement (70).

12. Analyseur selon l'une quelconque des revendications précédentes, dans lequel les moyens (160, 162, 164) définissant la piste sont agencés avec une forme courbe de manière à définir une piste courbe.

13. Procédé pour laver un élément de test incubé (K) comprenant les étapes consistant à :
charger un élément de test dans un premier poste (20) à l'intérieur d'un incubateur,
éjecter de l'incubateur un élément de test chargé, dans un second poste (80), à une certaine distance du premier poste,
saisir l'élément de test éjecté moyennant l'insertion d'une plaque (110, 120, 122, 124, 128, 130) dans le trajet de l'élément de test éjecté,
déplacer l'élément saisie jusqu'à un poste de lavage (70) par déplacement de la plaque et de son élément de test jusqu'au poste de lavage,
laver l'élément de test déplacé, dans le poste de lavage, et
ramener l'élément lavé à l'incubateur par retour de la plaque et de son élément de test dans le premier poste, et
réintroduire l'élément lavé dans l'incubateur.
